# EUROPÄISCHE PATENTSCHRIFT

(11) **EP 0 315 862 B1**
(45) Veröffentlichungstag und Bekanntmachung des Hinweises auf die Patenterteilung: **13.04.1994**
(21) Anmeldenummer: 88118102.8
(22) Anmeldetag: 31.10.1988
(51) Int. Cl.: C07C 217/78, C07C 323/36

(54) **Am aromatischen Kern Fluor enthaltende Fluormethoxy- und Fluormethylthioaminobenzole und deren Herstellung**
Aromatically fluorinated fluoromethoxy- and fluoromethylthio-amino benzenes and their preparation
Fluorométhoxy- et fluorométhylthioaminobenzènes fluorés sur le noyau aromatique et leur préparation

(30) Priorität: 09.11.1987 DE 3737985
(43) Veröffentlichungstag der Anmeldung: 17.05.1989
(73) Patentinhaber: BAYER AG, 51368 Leverkusen (DE)
(72) Erfinder: Marhold, Albrecht, Dr., D-5090 Leverkusen 1 (DE)

(56) Entgegenhaltungen:
- EP-A- 0 248 746
- EP-A- 0 318 704
- JP-A-62 223 158
- JP-A-62 298 562
- US-A- 4 044 132
- CHEMICAL ABSTRACTS, Band 106, Nr. 19, 11. Mai 1987, Seite 651, Zusammenfassung Nr. 156076x, Columbus, Ohio, US
- JOURNAL OF ORGANIC CHEMISTRY, Band 44, Nr. 16, 3. August 1979, Seiten 2907-2910, American Chemical Society, Columbus, Ohio, US; A.E. FEIRING: "Chemistry in hydrogen fluoride. 7. A novel synthesis of aryl trifluoromethyl ethers"

## Beschreibung

Die vorliegende Erfindung betrifft neue, am aromatischen Kern Fluor enthaltende Fluormethoxy-aminobenzole und neue, am aromatischen Kern Fluor enthaltende Fluormethylthio-aminobenzole, sowie Verfahren zu deren Herstellung.

Trifluormethoxy-aminobenzole können aus Aminophenolen, Tetrachlormethan und Fluorwasserstoff erhalten werden (J. Org. Chem. 44, 2907 (1979)). Der Einsatz von fluorierten Aminophenolen ist dort nicht beschrieben. Trifluormethoxyverbindungen lassen sich nitrieren und die erhaltenen Nitroverbindungen reduzieren, wobei Trifluormethoxy-aniline entstehen (J. Org. Chem. 29, 1 (1964)). Das Verfahren ist für kernfluorierte Trifluormethoxyverbindungen jedoch nicht bekannt, im Gegensatz dazu aber Nitroverbindungen von kernchlorierten Trifluormethoxybenzolen (Zh. Obshch. Khim. 31, 915 bis 924 (1961)). Der Halogenaustausch (Chlor als Kernsubstituent gegen Flour) ist jedoch an solchen Verbindungen nicht beschrieben. Dies trifft ebenso für die Trifluormethylthiobenzole zu.

Trifluormethylthiobenzole können nach zahlreichen Methoden hergestellt werden, die allerdings teilweise sehr speziellen Charakter haben und nicht auf Thiobenzole übertragbar sind, die andere Substituenten im Phenylteil enthalten, beispielsweise Nitro- oder Aminogruppen (siehe J. Org. Chem. 41, 1644 (1964) und Houben-Weyl, Methoden der organischen Chemie, Band E4, Seite 633 ff).

Aus der JP-A 62-223 158 sind die Verbindungen 4-Amino-2,5-difluor- und 4-Amino-2,3-difluor-trifluormethoxybenzol bekannt, aus der US-A 4 044 132 die Verbindung 5-Amino-2-fluor-trifluormethylthiobenzol, aus der EP-A 248 746 die Verbindung 5-Amino-2-fluor-trifluormethoxybenzol und aus C.A. 106, 156 076 x ebenfalls die Verbindung 4-Amino-2,3-difluor-trifluormethoxybenzol. Gemäß J. Org. Chem. 44 (16) 2907-2910 (1979) wurden einige Amino-trifluormethoxybenzole hergestellt, jedoch keine am aromatischen Kern Fluoratome enthaltende Aminotrifluormethoxybenzole.

In J. Org. Chem. 39 (12) 1758-61 (1974), J. Med. Chem. 12, 196-197 (1969) und US-P 3 900 519 wird die Herstellung von Fluor-anilinen, Fluor-chlor-anilinen bzw. Fluor-alkyl-anilinen beschrieben, nicht jedoch von Fluor-, Amino- und Trifluormethoxy- oder Trifluormethylthiogruppen enthaltenden Aromaten.

Die am aromatischen Kern Fluor enthaltenden Fluormethoxy-aminobenzole und Fluormethylthio-aminobenzole der vorliegenden Erfindung entsprechen der Formel (I) 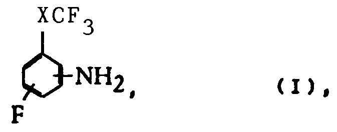
in der
- X: für Sauerstoff oder Schwefel,
ausgenommen 5-Amino-2-fluor-trifluormethoxybenzol und 5-Amino-2-fluor-trifluormethylthiobenzol.

Bevorzugte Verbindungen der Formel (I) sind:
2-Fluor-4-amino-trifluormethoxy-benzol, 3-Fluor-4-amino-trifluormethoxy-benzol, 3-Amino-4-fluor-trifluormethoxybenzol, 2-Amino-4-fluor-trifluormethoxy-benzol, 2-Amino-5-fluor-trifluormethoxy-benzol, 2-Fluor-4-amino-trifluormethyl-thiobenzol.

Ein allgemein anwendbares Verfahren zur Herstellung von Verbindungen der Formel (I) 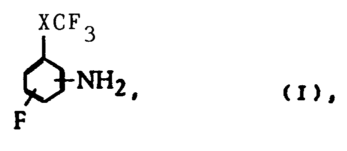
in der
- X: für Sauerstoff oder Schwefel,
ausgenommen ist die Verbindung 5-Amino-2-fluor-trifluormethyltiobenzol,
ist dadurch gekennzeichnet, daß man Verbindungen der Formel (II) 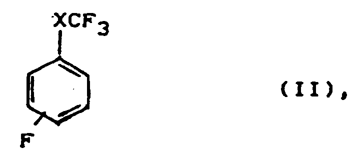
in der
- X: die bei Formel (I) angegebene Bedeutung hat,
nitriert und die so erhaltenen Nitroverbindungen reduziert.

Die in dieses Verfahren einzusetzenden, am aromatischen Kern Fluor enthaltenden Fluormethoxy- und Fluormethylthio-benzole der Formel (II) sind bekannt (siehe J. Org. Chem. 29, 1 (1964)) oder können analog dazu hergestellt werden.

Die Nitrierung kann mit üblichen Nitriermitteln durchgeführt werden, beispielsweise mit Gemischen aus Salpeter- und Schwefelsäure. Die Temperatur kann dabei beispielsweise im Bereich von 0 bis 80°C liegen, vorzugsweise liegt sie bei 20 bis 50°C. Das Nitriermittel kann man beispielsweise in Mengen einsetzen, bei denen im Reaktionsgemisch 0,8 bis 1,5 Mole Nitrieragenz pro Mol Ausgangsverbindung gebildet werden. Vorzugsweise läßt man sich 1 bis 1,1 Mol Nitrieragenz pro Mol Ausgangsverbindung bilden Die Nitrierung kann gegebenenfalls in Gegenwart eines inerten organischen Lösungsmittels durchgeführt werden. Geeignet ist beispielsweise Methylenchlorid.

Die daran anschließende Reduktion kann chemisch, d.h. beispielsweise mit reduzierend wirkenden Metallen oder Metallsalzen durchgeführt werden. Geeignet sind beispielsweise Eisen, Zink, Zinn, Zinn(II)chlorid und Titan(III)chlorid. Derartige Reduktionsmittel werden vorzugsweise in der stöchiometrisch erforderlichen Menge eingesetzt. Für eine derartige Reduktion können die Nitroverbindungen z.B. so eingesetzt werden, wie sie bei der Nitrierung anfallen oder danach isoliert werden. Die Reduktion kann man auch katalytisch mit Wasserstoff durchführen, wobei z.B. Katalysatoren eingesetzt werden können, die Metalle enthalten oder daraus bestehen.

Geeignet sind beispielsweise die Metalle der VIII. Nebengruppen des Periodischen Systems der Elemente, insbesondere Palladium, Platin und Nickel. Die Metalle können in elementarer Form oder in Form von Verbindungen vorliegen, sowie in besonders aktivierten Formen, z.B. in Form von Raney-Metallen oder aufgebracht als Metall oder Metallverbindung auf Trägermaterialien. Bevorzugt ist Raney-Nickel, Palladium auf Kohle und Palladium auf Aluminiumoxid.

Vorzugsweise wird die katalytische Reduktion in Gegenwart eines Lösungsmittels durchgeführt. Geeignet sind beispielsweise Alkohole und Ether, wie Methanol, Ethanol und Tetrahydrofuran. Die katalytische Reduktion kann beispielsweise bei Temperaturen im Bereich 0 bis 80°C und beispielsweise bei Wasserstoffdrucken im Bereich 1 bis 100 bar durchgeführt werden. Überschüsse an Wasserstoff sind im allgemeinen nicht kritisch.

Für die katalytische Reduktion werden vorzugsweise säurefreie Nitroverbindungen eingesetzt. Gegebenenfalls sind letztere also von Säuren zu befreien, z.B. durch Waschen mit Wasser oder Neutralisation mit einer Base.

Die Aufarbeitung des nach der chemischen Reduktion oder der katalytischen Hydrierung vorliegenden Reaktionsgemisches kann beispielsweise so erfolgen, daß man zunächst gegebenenfalls vorhandene feste Bestandteile abfiltriert und das Filtrat, gegebenenfalls nach einer Wäsche mit Wasser, destilliert. Falls als Reaktionsprodukt ein Gemisch von Isomeren anfällt, kann man dieses durch Feindestillation trennen.

Verbindungen der Formel (I) mit Fluor in o- und/oder p-Stellung zur Aminogruppe kann man auch herstellen, indem man Verbindungen der Formel (III) 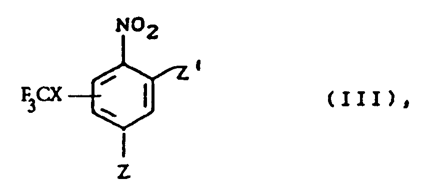
in der
- X: für Sauerstoff oder Schwefel,
und
mindestens eines der Symbole Z und Z' für Chlor und das andere für Chlor oder Wasserstoff steht,
mit einen Fluorierungsmittel (z.B. Kaliumfluorid) in Gegenwart eines polaren, aprotischen Lösungsmittels (z.B. N-Methylpyrrolidon, Dimethylsulfoxid oder Tetramethylensulfon) umsetzt, wobei am aromatischen Kern vorhandenes Chlor durch Fluor ersetzt wird, und anschließend eine Reduktion durchführt, wobei die Nitrogruppe in eine Aminogruppe überführt wird. Vorzugsweise führt man diese Reaktion in Gegenwart von Katalysatoren (z.B. quarternäre Ammoniumsalze oder Kronenether) durch, man kann aber häufig auch ohne Katalysatoren befriedigende Ergebnisse erhalten.

Verbindungen der Formel (III) sind bekannt (siehe Houben-Weyl, Band E4, S. 633 ff und Zh. obshch. Khim. 31, 915-924 (1961)).

Pro Äquivalent in der Verbindung der Formel (III) gegen Fluor auszutauschendes Chlor kann man beispielsweise 0,5 bis 3 Mole Fluorierungsmittel einsetzen. Vorzugsweise beträgt diese Menge von 1,2 bis 1,5 Mole. Das Lösungsmittel wird vorzugsweise mindestens in einer solchen Menge eingesetzt, daß ein gut rührbares Reaktionsgemisch vorliegt. Größere Mengen an Lösungsmittel stören nicht. Katalysatoren können gegebenenfalls z.B. in einer Menge von 0,01 bis 0,5 Mol-% eingesetzt werden.

Geeignete Temperaturen für die Umsetzung mit einem Fluorierungsmittel sind beispielsweise solche im Bereich von 160 bis 230°C. Bevorzugte Temperaturen sind solche von 180 bis 210°C. Vorzugsweise führt man die Umsetzung in möglichst wasserfreiem Milieu durch. Dies kann man beispielsweise dadurch erreichen, daß man als letzte Komponente die Verbindung der Formel (III) in sorgfältig getrockneter Form einsetzt und aus den vorher zusammengegebenen sonstigen Komponenten eine kleine Menge Lösungsmittel zusammen mit gegebenenfalls vorhandenem Wasser abdestilliert.

Nach Beendigung der Reaktion, die normalerweise zwischen 1 und 12 Stunden Reaktionszeit erfordert, können im Reaktionsgemisch vorliegende Feststoffe und gegebenenfalls ganz oder teilweise das Lösungsmittel entfernt werden.

Die anschließende Reduktion der Nitro- zur Aminogruppe und die Aufarbeitung des dann vorliegenden Reaktionsgemisches kann so erfolgen, wie zuvor beim allgemein anwendbaren Verfahren zur Herstellung der erfindungsgemäßen Verbindungen beschrieben worden ist.

Verbindungen der Formel (I) mit X = Sauerstoff können auch hergestellt werden, indem man Verbindungen der Formel (IV) 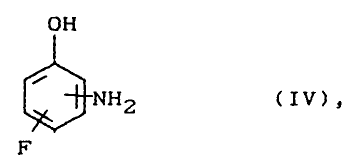
mit Tetrachlorkohlenstoff in Gegenwart von Fluorwasserstoff umsetzt, wobei die OH-Gruppe in eine CF₃O-Gruppe
überführt wird.

Verbindungen der Formel (IV) sind bekannt (siehe FR-PS 2 446 805).

Pro Mol der jeweiligen Verbindung der Formel (IV) kann man beispielsweise 1 bis 10 Mole Tetrachlorkohlenstoff und 5 bis 30 Mole Fluorwasserstoff einsetzen. Auch größere Überschüsse von Tetrachlorkohlenstoff und Fluorwasserstoff stören im allgemeinen nicht. Geeignete Reaktionstemperaturen sind beispielsweise solche im Bereich von 100 bis 150°C. Dieses Verfahren führt man vorzugsweise unter Druck durch, beispielsweise indem man das entstehende Chlorwasserstoffgas erst oberhalb eines bestimmten Druckes entspannt. Dieser Druck kann beispielsweise bei 18 bis 60 bar liegen. Gegebenenfalls kann man zusätzlich ein inertes Gas aufdrücken, z.B. 1 bis 20 bar Stickstoff. Es ist vorteilhaft, während der Reaktion gut zu rühren.

Die Aufarbeitung des Reaktionsgemisches kann man beispielsweise so durchführen, daß man das Reaktionsgemisch auf Raumtemperatur abkühlt, entspannt, überschüssigen Fluorwasserstoff und überschüssigen Tetrachlorkohlenstoff beispielsweise bei Temperaturen bis 80°C abdestilliert, den Rückstand auf Eiswasser gibt, mit Natronlauge alkalisch stellt, die organische Phase mit Dichlormethan extrahiert und nach dem Trocknen einer Feindestillation unterwirft.

Die erfindungsgemäßen, am aromatischen Kern Fluor enthaltenden Fluormethoxy- und Fluormethylthio-aminobenzole der Formel (I) sind wertvolle Zwischenprodukte. Beispielsweise kann man sie mit Acrylnitril unter Diazotierungsbedingungen umsetzen, so α-Chlor-β-phenyl-propionnitrile erhalten, deren Phenylanteil (mit Ausnahme der NH₂-Gruppe) in gleicher Weise substituiert ist wie im jeweils eingesetzten erfindungsgemäßen Produkt, diese durch Dehydrohalogenierung in das entsprechende Zimtsäurenitril überführen und daraus durch Umsetzung mit Sulfonylmethylisocyaniden 3-Cyano-4-phenyl-pyrrole gewinnen, deren Phenylteil (mit Ausnahme der NH₂-Gruppe) in gleicher Weise substituiert ist, wie im jeweils eingesetzten erfindungsgemäßen Produkt. Derartige 3-Cyano-4-phenyl-pyrrole weisen eine besonders gute Wirksamkeit als Fungizide auf, insbesondere zum Schutz von Pflanzen gegen Plasmodiophoromycetes, Oomycetes, Chrytridiomycetes, Zygomycetes, Ascomycetes, Basidiomycetes und Deuteromycetes. Solche 3-Cyano-4-phenyl-pyrrole, deren Herstellung und deren Verwendung als Schädlingsbekämpfungsmittel sind von separaten eigenen Patentanmeldungen umfaßt ( = europäische Patentanmeldung EP-A-318 704).

### Beispiele

### Beispiel 1

In einer Fluorierungsapparatur wurden 100 g 2-Fluor-4-hydroxy-anilin, 500 ml wasserfreier Fluorwasserstoff und 500 ml Tetrachlormethan vorgelegt und 3 bar Stickstoff aufgedrückt. Anschließend wurde unter Rühren mit 600 U/min für 7 Stunden auf 120°C erhitzt und der entstehende Chlorwasserstoff bei 38 bar kontinuierlich entspannt. Nach Ende der Reaktion wurde das Gemisch auf 20°C gekühlt, entspannt und überschüssiger Fluorwasserstoff zusammen mit Tetrachlorkohlenstoff bis 80°C abdestilliert. Der Rückstand wurde nach Abkühlen auf 500 ml Eiswasser gegossen und unter Kühlung mit Natronlauge alkalisch gestellt. Anschließend wurde die organische Phase mit Dichlormethan extrahiert, getrocknet und im Vakuum einer Feindestillation unterworfen. Bei einem Siedepunkt von 68 bis 69°C bei 20 mbar gingen 53 g 3-Fluor-4-amino-trifluormethoxy-benzol über.

### Beispiel 2

a) In einer Rührapparatur wurden 90 g 4-Trifluormethoxy-fluorbenzol vorgelegt und bei 10 bis 15°C 105 g Mischsäure (33 Gew.-% Salpetersäure, 67 Gew.-% Schwefelsäure) zugetropft. Es wurde für 2 Stunden bei 20°C nachgerührt und dann auf Eis gegossen. Nach Abtrennung der organischen Phase mit Hilfe von Dichlormethan wurde die Lösung getrocknet und destilliert. Im Siedebereich von 90 bis 94°C bei 15 mbar ging ein Gemisch bestehend aus 43 Gew.-% 3-Nitro-4-fluor-trifluormethoxy-benzol und 54 Gew.-% 2-Nitro-4-fluor-trifluormethoxybenzol in einer Menge von 112 g über.
b) In einer Hydrierapparatur wurden 112 g der gemäß a) erhaltenen Nitroverbindungen in 450 ml Methanol zusammen mit 12 g Raney-Nickel vorgelegt und mit 30 bar Wasserstoff bei 25 bis 45°C hydriert. Nach Abkühlen und Entspannen wurde das Gemisch filtriert und anschließend das Filtrat einer Feindestillation unterworfen. Im Siedebereich 58 bis 60°C bei 14 mbar gingen 32 g 2-Amino-4-fluor-trifluormethoxy-benzol über und nach einem Zwischenlauf bei 64 bis 65°C und 15 mbar 28 g 3-Amino-4-fluor-trifluormethoxy-benzol.

### Beispiel 3

a) Analog Beispiel 2 wurden 83 g 3-Trifluormethoxyfluorbenzol mit 100 g Nitriersäure bei 15°C nitriert. Die Reaktionsmischung bestand nach analoger Aufarbeitung zu 78 Gew.-% aus 3-Fluor-4-nitro-trifluormethoxy-benzol und zu 20 Gew.-% aus 3-Fluor-6-nitro-trifluormethoxy-benzol. Die Gesamtausbeute betrug 99 g.
b) In einer Hydrierapparatur wurden die gemäß a) hergestellten 99 g Nitroverbindungen in 350 ml Methanol in Gegenwart von 10 g Raney-Nickel bei 25 bis 40°C mit 30 bis 50 bar Wasserstoff hydriert. Nach Ende der Wasserstoffaufnahme wurde abkühlt und entspannt, die festen Bestandteile des Reaktionsgemisches abfiltriert und danach das Lösungsmittel durch Destillation entfernt. Die Feindestillation des Rückstandes lieferte im Siedebereich von 53 bis 54°C bei 10 mbar 5 g 3-Fluor-6-amino-trifluormethoxy-benzol und nach einem Zwischenlauf 38 g 3-Fluor-4-amino-trifluormethoxy-benzol in einem Siedebereich von 56 bis 57°C bei 10 mbar.

## Patentansprüche

1. Am aromatischen Kern Fluor enthaltende Fluormethoxy-aminobenzole und Fluormethylthio-aminobenzole der Formel 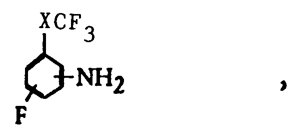 in der
X für Sauerstoff oder Schwefel,
ausgenommen 5-Amino-2-fluor-trifluormethoxybenzol und 5-Amino-2-fluor-trifluormethyl-thiobenzol.

2. Verbindungen gemäß Anspruch 1, dadurch gekennzeichnet, daß es sich um 2-Fluor-4-amino-trifluormethoxy-benzol, 3-Fluor-4-amino-trifluormethoxy-benzol, 3-Amino-4-fluor-trifluormethoxy-benzol, 2-Amino-4-fluor-trifluormethoxy-benzol, 2-Amino-5-fluor-trifluormethoxy-benzol und 2-Fluor-4-amino-trifluormethyl-thiobenzol handelte.

3. Verfahren zur Herstellung von Verbindungen der Formel 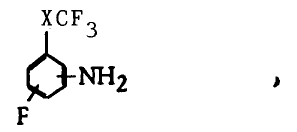 in der
X für Sauerstoff oder Schwefel,
ausgenommen ist die Verbindung 5-Amino-2-fluor-trifluormethylthiobenzol,
dadurch gekennzeichnet, daß man Verbindungen der Formel 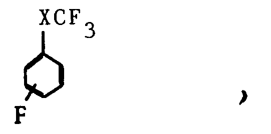 in der
X die oben angegebene Bedeutung hat,
nitriert und die so erhaltenen Nitroverbindungen reduziert.

4. Verfahren zur Herstellung von Verbindungen der Formel 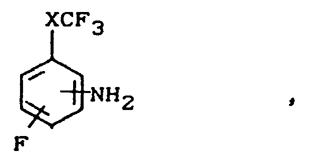 in der
X für Sauerstoff oder Schwefel und
das oder die Fluoratome in o- und/oder p-Stellung zur Aminogruppe angeordnet sind, ausgenommen ist die Verbindung 5-Amino-2-fluor-trifluormethoxybenzol,
dadurch gekennzeichnet, daß man Verbindungen der Formel 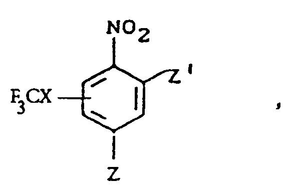 in der
X für Sauerstoff oder Schwefel,
und
mindestens eines der Symbole Z und Z' für Chlor und das andere für Chlor oder Wasserstoff steht,
mit einem Fluorierungsmittel in Gegenwart eines polaren, aprotischen Lösungsmittels umsetzt, wobei am aromatischen Kern vorhandenes Chlor durch Fluor ersetzt wird, und anschließend eine Reduktion durchführt, wobei die Nitrogruppe in eine Aminogruppe überführt wird.

5. Verfahren zur Herstellung von Verbindungen der Formel 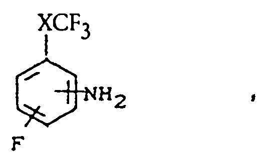 in der
X fur Sauerstoff steht,
dadurch gekennzeichnet, daß man Verbindungen der Formel 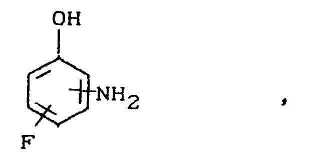 mit Tetrachlorkohlenstoff in Gegenwart von Fluorwasserstoff umsetzt, wobei die OH-Gruppe in eine CF₃O-Gruppe überführt wird.

## Claims

1. Fluoromethoxy-aminobenzenes and fluoromethylthioaminobenzenes containing fluorine in the aromatic nucleus of the formula 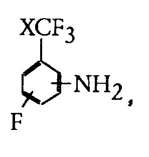 in which
X represents oxygen or sulphur,
with the exception of 5-amino-2-fluoro-trifluoromethoxybenzene and 5-amino-2-fluoro-trifluoromethylthiobenzene.

2. Compounds according to Claim 1, characterized in that they are 2-fluoro-4-amino-trifluoromethoxybenzene, 3-fluoro-4-amino-trifluoromethoxy-benzene, 3-amino-4-fluoro-trifluoromethoxy-benzene, 2-amino-4-fluoro-trifluoromethoxy-benzene, 2-amino-5-fluorotrifluoromethoxy-benzene and 2-fluoro-4-aminotrifluoromethyl-thiobenzene.

3. Process for the preparation of compounds of the formula 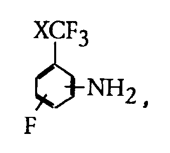 in which
X represents oxygen or sulphur,
with the exception of the compound 5-amino-2-fluorotrifluoromethylthiobenzene,
characterized in that compounds of the formula 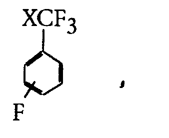 in which
X has the abovementioned meaning, are nitrated and the nitro compounds thus obtained are reduced.

4. Process for the preparation of compounds of the formula 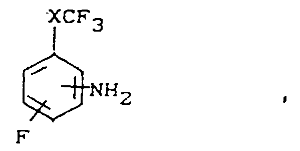 in which
X represents oxygen or sulphur and
the fluorine atom or atoms are arranged in the o-and/or p-position to the amino group, with the exception of the compound 5-amino-2-fluorotrifluoromethoxybenzene,
characterized in that compounds of the formula 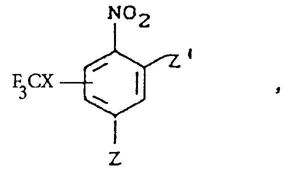 in which
X represents oxygen or sulphur,
and at least one of the symbols Z and Z' represents chlorine and the other represents chlorine or hydrogen,
are reacted with a fluorinating agent in the presence of a polar, aprotic solvent, where chlorine present in the aromatic nucleus is replaced by fluorine, and a reduction is subsequently carried out in which the nitro group is converted into an amino group.

5. Process for the preparation of compounds of the formula 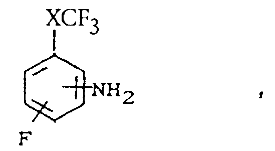 in which
X represents oxygen,
characterized in that compounds of the formula 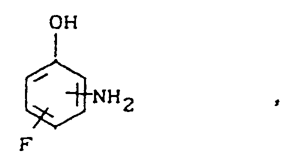 are reacted with carbon tetrachloride in the presence of hydrogen fluoride, the OH group being converted into a CF₃O group.

## Revendications

1. Fluorométhoxyaminobenzènes et fluorométhylthioaminobenzènes fluorés sur le noyau aromatique de formule 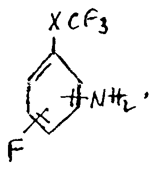 dans laquelle
X est de l'oxygène ou du soufre,
excepté le 5-amino-2-fluorotrifluorométhoxybenzène et le 5-amino-2-fluorotrifluorométhylthiobenzène.

2. Composés suivant la revendication 1, caractérisés en ce qu'ils comprennent le 2-fluoro-4-aminotrifluorométhoxybenzène, le 3-fluoro-4-aminotrifluorométhoxybenzène, le 3-amino-4-fluorotrifluorométhoxybenzène, le 2-amino-4-fluorotrifluorométhoxybenzène, le 2-amino-5-fluorotrifluorométhoxybenzène et le 2-fluoro-4-aminotrifluorométhylthiobenzène.

3. Procédé de production de composés de formule 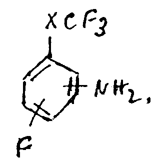 dans laquelle
X est de l'oxygène ou du soufre,
excepté le composé 5-amino-2-fluorotrifluorométhylthiobenzène,
caractérisé en ce qu'on effectue la nitration de composés de formule 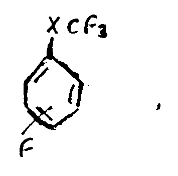 dans laquelle
X a la définition indiquée ci-dessus
et on réduit les composés nitrés ainsi obtenus.

4. Procédé de production de composés de formule 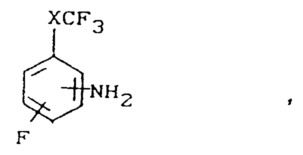 dans laquelle
X est de l'oxygène ou du soufre et,
l'atome ou les atomes de fluor sont en position ortho et/ou en position para par rapport au groupe amino, excepté le composé 5-amino-2-fluorotrifluorométhoxybenzène,
caractérisé en ce qu'on fait réagir des composés de formule 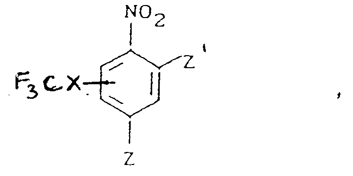 dans laquelle
X représente de l'oxygène ou du soufre,
et
l'un au moins des symboles Z et Z' représente du chlore et l'autre représente du chlore ou de l'hydrogène,
avec un agent de fluoration en présence d'un solvant aprotique polaire, pour remplacer du chlore présent sur le noyau aromatique par du fluor, puis on effectue une réduction en transformant le groupe nitro en un groupe amino.

5. Procédé de production de composés de formule 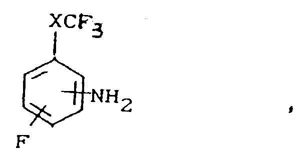 dans laquelle
X représente de l'oxygène,
caractérisé en ce qu'on fait réagir des composés de formule 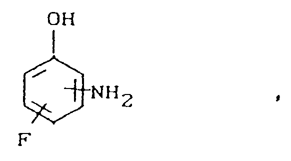 avec du tétrachlorure de carbone en présence de fluorure d'hydrogène pour transformer le groupe OH en un groupe CF₃O.
